# EUROPEAN PATENT SPECIFICATION

(11) **EP 4 112 064 B1**
(45) Date of publication and mention of the grant of the patent: **24.07.2024**
(21) Application number: 22181397.5
(22) Date of filing: 27.06.2022
(51) Int. Cl.: A61K 31/733, A61K 31/122, A61K 31/198, A61K 31/593, A61K 31/702, A61K 31/7048, A61K 35/741, A61K 36/15, A61K 36/185, A61K 36/42, A61K 8/67, A61K 8/9789, A61K 8/44, A61K 8/49, A61K 8/73, A61K 8/99, A61P 29/00, A23L 33/10, A61Q 19/00, A61Q 19/08, A61K 35/744, A61K 35/747, A61K 36/07, A61K 31/366, A61K 31/202

(54) **SKIN CARE NUTRACEUTICAL COMPOSITION**
HAUTPFLEGEZUSAMMENSETZUNG
COMPOSITION NUTRACEUTIQUE DE SOIN DE LA PEAU

(30) Priority: 28.06.2021 EP 21182180
(43) Date of publication of application: 04.01.2023
(73) Proprietor: ASC REGENITY Limited, London W1C 2PE (GB)
(72) Inventor: GIRI, Shibashish, 04109 Leipzig (DE)
(74) Representative: Yes My Patent

(56) References cited:
- FR-A1- 3 001 633
- JP-A- 2006 008 566
- US-A1- 2015 374 769
- US-A1- 2018 263 944
- US-A1- 2021 038 656
- ANONYMOUS: "Natural Botanical Extracts", CARRUBBA, 26 February 2007 (2007-02-26), pages 1 - 247, XP055151596, Retrieved from the Internet <URL:http://www.carrubba.com/pdf/Carrubba_Botanical_Guide_r.pdf> [retrieved on 20141107]

## Description

### Field of the Invention

The invention generally relates to a nutraceutical composition for skin care and more specifically the invention relates to a nutraceutical composition for skin care comprising probiotics.

### Background of the Invention

Most human prefer to look young, thus they do not hesitate to spend on beauty products. In recent years this need has extended to supply of beauty enhancing food supplements area to meet the needs. Further more people are aware of the benefits of seeking "beauty from within" and are increasingly adopting higher price "cosmeceuticals". There is an increasing interest particularly in maintaining the health of their skin.

Beauty supplement sales have grown exponentially in recent past and expected to grow by 5% every year in near future. Therefore, beauty supplements can no longer be considered a niche market as more consumers become convinced of the benefits of seeking beauty through nutrition. Research showed that many women and celebrities are using beauty supplements to enhance skin care or would consider using them in the future. Most of them contain vitamins, antioxidants and omega-3 fatty acids.

Those in primary need are people between the ages of 35 and 50 years old who would like to protect against the visible signs of skin ageing. Those in secondary need are people of any age who have specific "problem" areas to address, most notably their skin, e.g. blemishes, redness, oily skin, etc.

A proper diet is a contributing factor in maintaining healthy skin. There are a variety of known dietary supplements which can affect human skin conditions. Oral administration of food supplements products can be beneficial for maintaining healthy skin and for treatment of skin conditions, but the result may reflect after a prolonged use or constant use for years.

Further certain skin cream can activate the endogenous stem cells of human facial skin for skin to give great look on facial skin but this is superficially and temporary solution. It is important to rebuild or create new facial skin cells internally inside of body in a natural way of facial skin regeneration like a skin of growing children. Special cellular and molecular regenerative factors are active in the skin of growing children. As we age those factors are remain less efficient, therefore aged facial skin looks poor complexion, cellular dehydration, fine lines and wrinkles.

Scientists have proposed various theories for the reason we age, including mitochondrial dysfunction, inflammation, DNA damage, cell senescence, and telomere reduction

Further it has been proposed in various theories for the reason of ageing, including mitochondrial dysfunction, inflammation, DNA damage, cell senescence, and telomere reduction in numerous innovations provided in prior art that are adapted to several methods, compositions, cosmetics have been developed and are already being used to hide or provide temporary solution to skin ageing problem. Even though these innovations may be suitable for the specific purposes to which they address, however, they would not be as suitable for the purposes of the present invention.

Numerous attempts have been made and several prior art devices are known for variety of holders for food products. Even though these innovations may be suitable for the specific purposes to which they address, however, they would not be as suitable for the purposes of the present invention.

For example, PCT patent application No. WO2020205528A1 to Rodolphe Barrangou et al. discloses about a probiotic nutraceutical compositions, comprising a probiotic bacterium in the presence of pomegranate extract, wherein the composition is used to cure a digestive disease, an inflammatory disease, cancer, or any combination thereof.

PCT patent application No. WO2020096992A1 to Jayamani Elamparithi et al. discloses about a method of manufacturing a probiotic product comprising an ellagitannin-enzyme-synthesizing (EES) microbe, comprising adding an ellagitannin composition to the probiotic product, where the EES microbe expresses one or more ellagitannin enzymes.

PCT patent application No. WO2014147280A1 to Francisco TOMÁS BARBERÁN et al. discloses about a method of preparation of probiotic compositions by micro-organisms of human gastrointestinal origin, that can produce urolithins.

U. S. Patent No. US9289375B2 to Christian Drapeau et al. discloses a Skin care compositions topically administered to the skin containing combinations of natural ingredients.

U. S. Published Patent Application No. US20140301994A1 to Andrew M. Klapper et al. discloses about an anhydrous mixture of biologically active probiotic bacteria for topical administration and methods of making thereof. The mixture of the invention is used for hydrating the skin, reducing and preventing fine lines and wrinkles, treating acne and decreasing skin inflammation.

U. S. Patent No. US8481299B2 to Audrey Gueniche discloses about a method directed to the cosmetic use of an effective amount of at least one probiotic microorganism especially from the genus Lactobacillus sp. and/or Bifidobacterium sp., of a fraction thereof and/or of a metabolite thereof, as an active agent for limiting, preventing or treating skin irritation and/or irritative skin disorders.

U. S. Published Patent Application No. US20160338979A1 to Chun-Ming Huang discloses about a topical probiotic composition for producing or maintaining skin microbiome balance comprising a topical probiotic composition that is capable of producing or maintaining skin microbiome balance. The composition can comprise a therapeutically effective amount or inhibiting effective amount of one or more microbiome balancing compounds.

Several inventions in the prior art are already being used to alleviate or reverse the skin ageing issues in human being, they are either use synthetic compounds in the food supplements or the food supplement is not efficient enough to give quick result on the skin care or as a topical composition. Furthermore, even though these innovations may be suitable for the specific purposes to which they address, accordingly, they would not be suitable for the purposes of the present invention as heretofore described. Thus, a solution which can rebuild or create new facial skin cells by nutrients produced internally inside our body in a natural way for facial skin regeneration to provide beautiful, youthful, elastic skin for a glowing face is needed.

### Summary of the Invention

The present invention discloses about a probiotic based skin care food or nutraceutical composition that rebuild or create new facial skin cells internally inside of body in a natural way of facial skin regeneration.

According to an aspect of the present invention, the probiotic-based skin care nutraceutical composition to kick start and activate cellular and molecular regenerative factors and able to create beautiful, youthful, elastic skin. The probiotic nutraceutical composition of the present invention is specially designed and mimics the function of the growing childrens' skin. Growing children do not need any cream or food supplements, their skin glow naturally. But as we age, our facial skin starts to lose its power to regenerate new facial skin cells. The Probiotic nutraceutical composition is the ultimate alternative of regenerative factors of skin of growing children.

According to another aspect of the present invention, the probiotic nutraceutical composition of the present invention activates facial endogenous stem cells internally along with cellular and molecular factors with efficiently in natural way of new facial skin regeneration.

According to another aspect of the present invention, the probiotic nutraceutical composition of the present invention creates new facial skin cells with three layers of tissue: the epidermis, dermis and hypodermis with youthful look.

According to another aspect of the present invention, the probiotic nutraceutical composition of the present invention contains much powerful ingredients, those are not only activating the telomerase enzyme in optimal level but also other necessary cellular and molecular factors to create optimal level of nitric oxide inside human body, repair the damages DNA which are mandatory for natural way of facial skin regeneration. Very recent and new millstone discovery: creation of new cellular mitochondria from damaged mitochondria, secrete collagen 17 in facial skin cells (without this collagen 17, stem cells activation is incomplete and not able to proliferate), after collagen synthesis, it is required to be proper organization of collagen in facial skin cells, Vitamin K2 has excellent role for proper organization of synthesized collagens in facial skin cells. Natto is the best sources of vitamin K2 but taste is very bad. Peoples hate to eat it. The probiotic nutraceutical composition contains vitamin K2 extract from Natto. Ingredients of the probiotic nutraceuticalcomposition stimulate glutathione enzyme level naturally and erase of free radicals of contaminated facial skin cells. Additionally, ingredients create new functional neurons and new blood vessels. New functional neurons give proper message to brain to deliver the necessary nutrients, component inside of human body. New blood vessels are like bridge, facilities to brings lot of regenerative factors to facial regions from other parts of body under guidance of human good mood brain. Gut microbes is considered as second brain. Human brain and intestinal microbes (second brain) always are in communication and can create happy facial skin appearance. The probiotic nutraceutical composition is specially designed to diminished age spots and skin imperfections and cerate great happy and healthy facial skin.

According to another aspect of the present invention, the skin care nutraceutical composition comprises Organic Inulin, premix of probiotics, L-Citrulline, pomegranate extracts, pine bark extract (very soluble grade 98%), organic beet root extract, L.Arginine, organic watermelon rind extract, vitamin K2 and vitamin D3.

According to another aspect of the present invention, the skin care nutraceutical composition comprises organic inulin, premix of probiotics, L-Citrulline, pomegranate extracts, pine bark extract (very soluble grade 98%), organic beet root extract, L-Arginine, organic watermelon rind extract, vitamin K2, vitamin D3, proferrin or lactoferrin, vitamin B3, vitamin B5 and vitamin B6.

According to another aspect of the present invention, the skin care nutraceutical composition comprises organic inulin, oligofructose, premix of probiotics, L-Citrulline, pomegranate extracts, pine bark extract (very soluble grade 98%), proferrin or lactoferrin, organic beet root extract, ashwagandha extract, vitamin C, L-Arginine, organic watermelon rind extract, vitamin B3, ZINC Bisglycinate, vitamin B6, vitamin B5, vitamin B9, vitamin K2, vitamin D3, L-Selenomethionine, and a flavor Silarom YUZU 1201304908.

In view of the foregoing, it is therefore an object of the present invention to provide a probiotic based skin care food or nutraceutical composition that kick start and activate cellular and molecular regenerative factors to rebuild or create new facial skin cells internally inside of body in a natural way of facial skin regeneration.

It is another objective of the present invention to provide a probiotic based skin care food or nutraceutical composition that activates facial endogenous stem cells internally along with cellular and molecular factors with efficiently in natural way of new facial skin regeneration.

It is another objective of the present invention to provide a probiotic based skin care food or nutraceutical composition that activates the telomerase enzyme in optimal level but also other necessary cellular and molecular factors to create optimal level of nitric oxide inside human body, repair the damages DNA which are mandatory for natural way of facial skin regeneration.

It is another objective of the present invention to provide a probiotic based skin care food or nutraceutical composition that create new cellular mitochondria from damaged mitochondria and secrete collagen 17 in facial skin cells.

It is another objective of the present invention to provide a probiotic based skin care food or nutraceutical composition that stimulate glutathione enzyme level naturally and erase of free radicals of contaminated facial skin cells.

It is another objective of the present invention to provide a probiotic based skin care food or nutraceutical composition that facilitate to create Urolithin A, which is a mitophagy activator which create new, healthy mitochondria from old or damaged mitochondria of facial skin cells.

It is another objective of the present invention to provide a probiotic based skin care food or nutraceutical composition that comprises human friendly intestinal microbes and a grow medium having pomegranate extract comprising Punicalagins, Ellagitannin, Punicic acid, Ellagitannis, wherein the microbes reaching inside human body to create an ambient environment for these friendly microbes to live and multiply in human body while producing Urolithin A in good quantity and in natural way to facilitate and speed up mitophagy in human cells that facilitate skin regeneration.

It is another objective of the present invention to provide a probiotic based skin care food or nutraceutical composition that naturally produces Urolithin A to get absorbed in human intestine and transport to facial region, thus the skin care nutraceutical composition acts as a skin progenitor.

It is another objective of the present invention to provide a probiotic based skin care food or nutraceutical composition that comprises probiotics selected from Lactobacillus acidophilus, Lactobacillus delbrueckii subsp. Bulgaricus, Streptococcus thermophilus, Lactococcus lactis ssp. Cremoris, Lactobacillus helveticus and a combination thereof in 20-30 billion CFUs (colony forming units), wherein these microbes are supplied with food supplements for them such as inulin and oligofructose to eat at different stages and different parts of our intestine thereby facilitating quick multiply of the number of friendly microbes inside the intestine.

It is another objective of the present invention to provide a probiotic based skin care food or nutraceutical composition that activates the telomerase enzyme in optimal level to boost production of hyaluronic acid and collagen.

It is another objective of the present invention to provide a probiotic based skin care food or nutraceutical composition that stimulates TGF-β3 on skin cells to generate scar free skin regeneration while reduce the secretion of TGF-β1 and TGF-β2, thereby facilitating skin regeneration at a faster rate to improve overall tone, texture, complexion and clarity of the skin.

It is another objective of the present invention to provide a probiotic based skin care food or nutraceutical composition that comprises Organic Inulin, premix of probiotics, L-Citrulline, pomegranate extracts, pine bark extract (very soluble grade 98%), organic beet root extract, L.Arginine, organic watermelon rind extract, vitamin K2 and vitamin D3.

Other features and aspects of the invention will become apparent from the following detailed description, which illustrate, by way of example, the features in accordance with embodiments of the invention.

### Brief Description of the Drawings

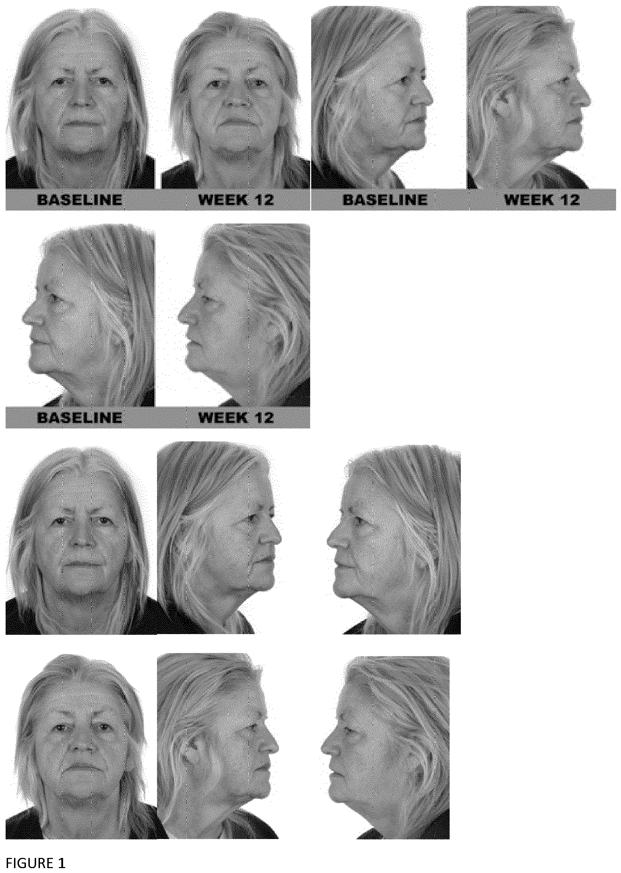

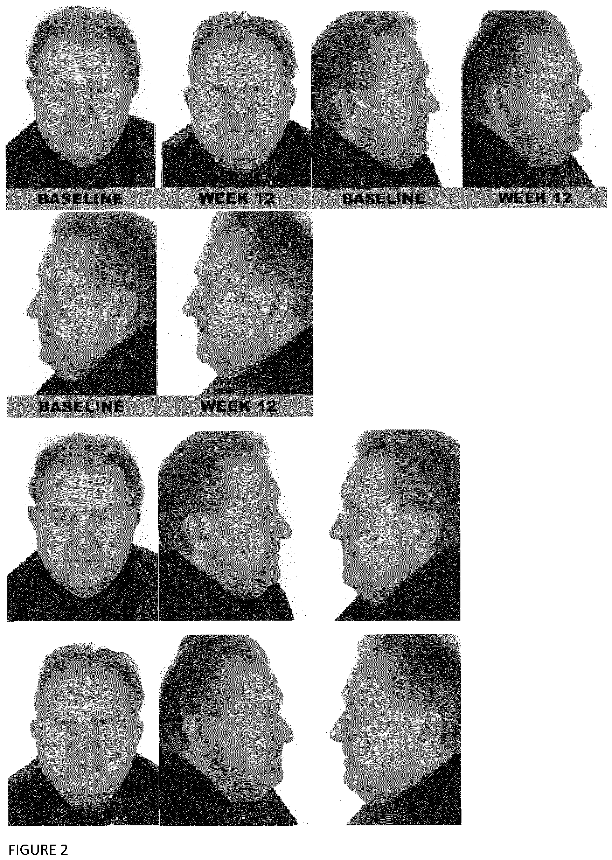

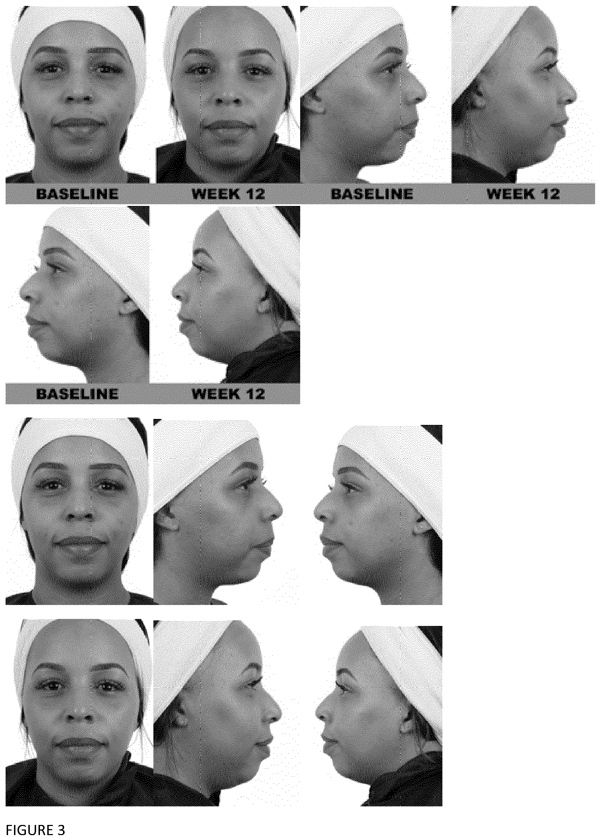

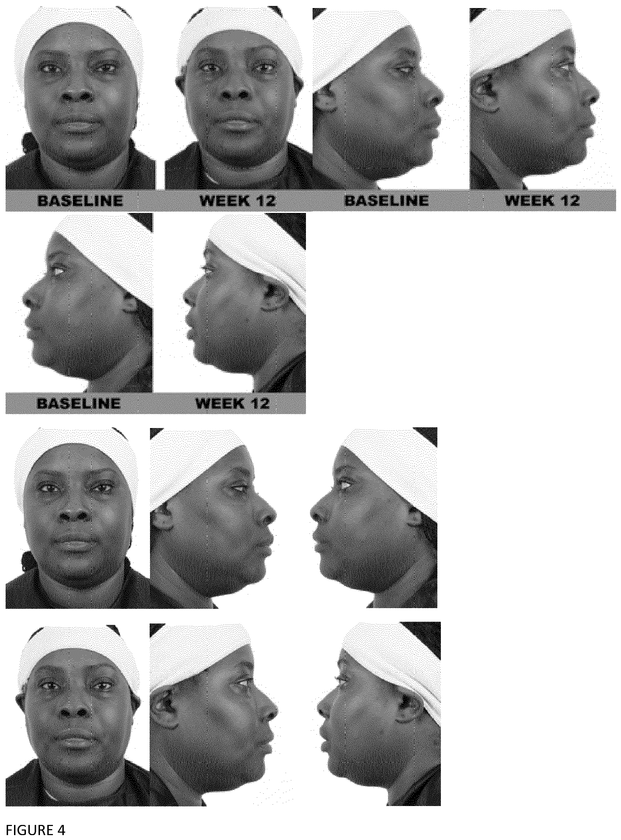

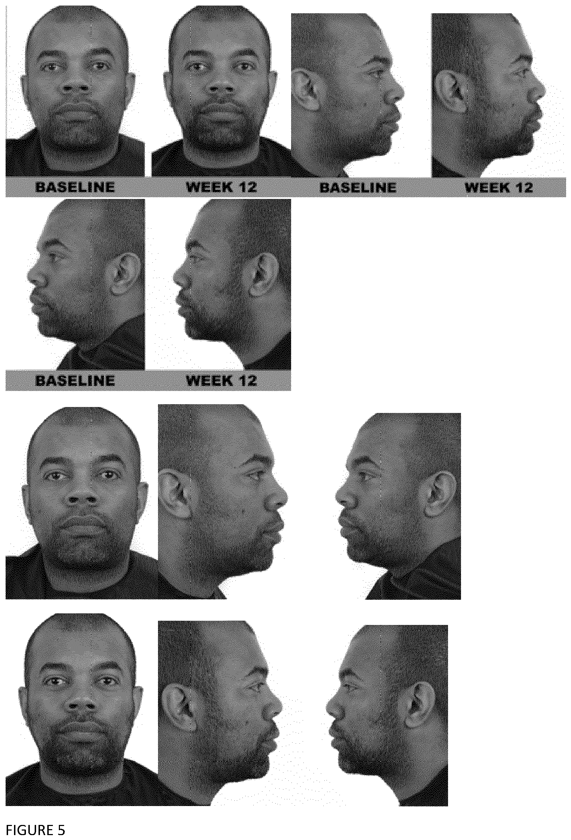

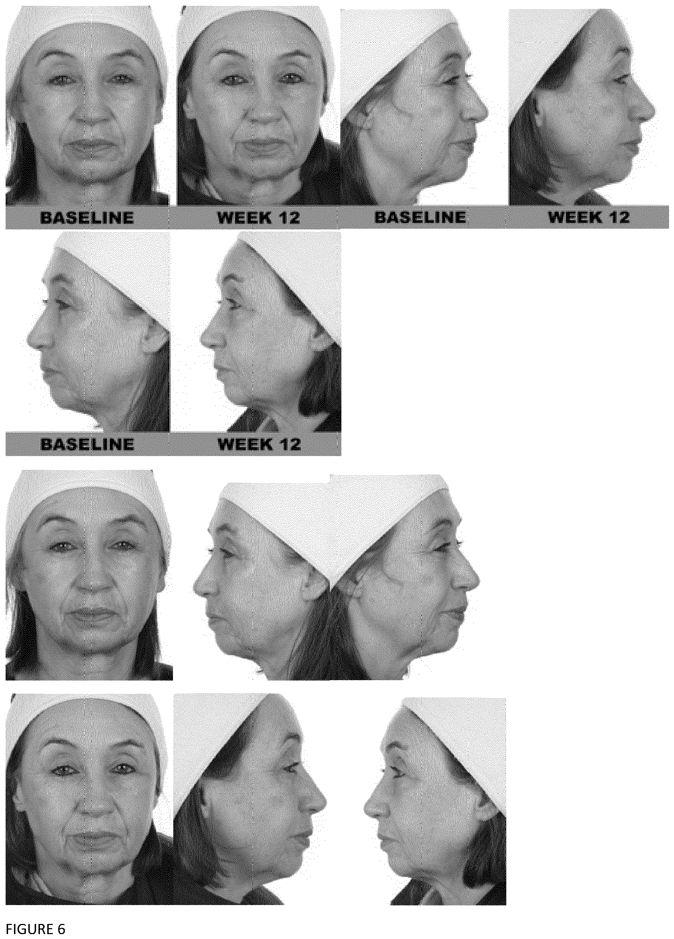

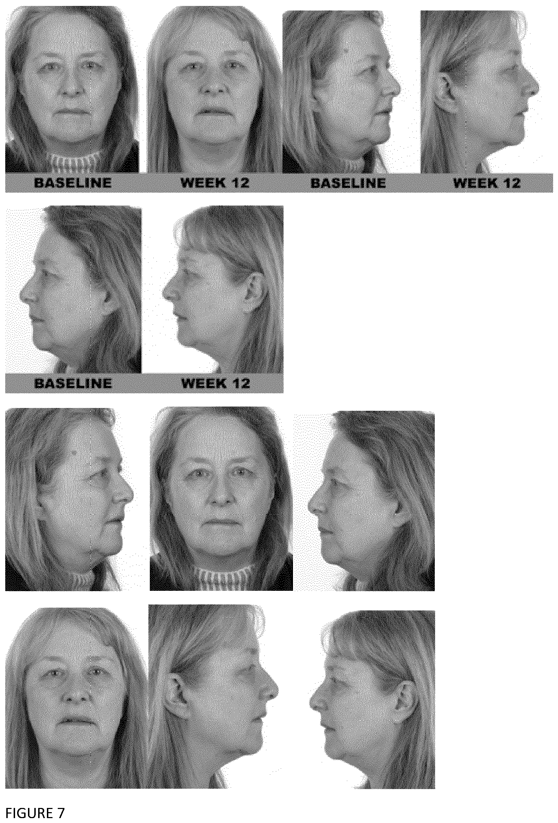

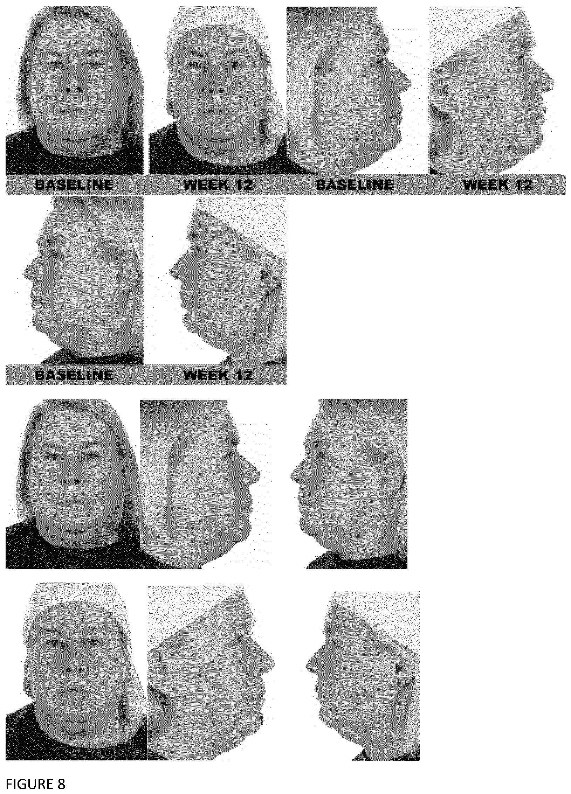

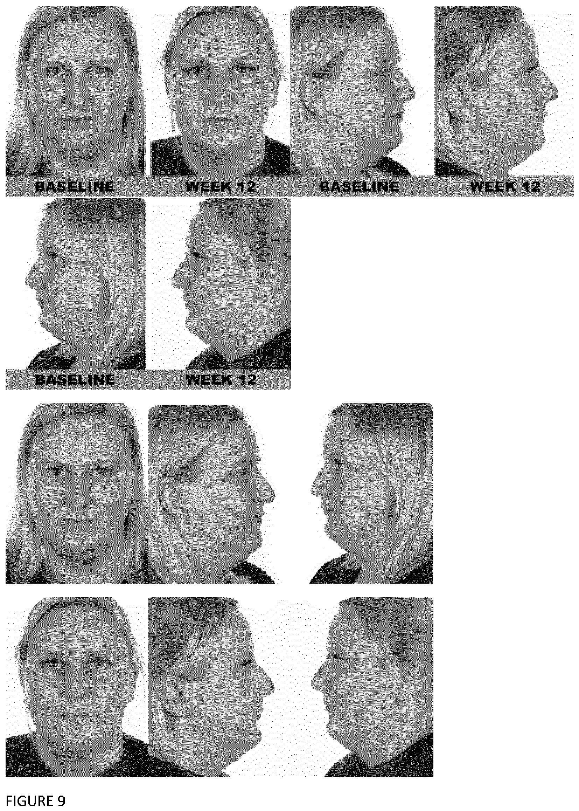

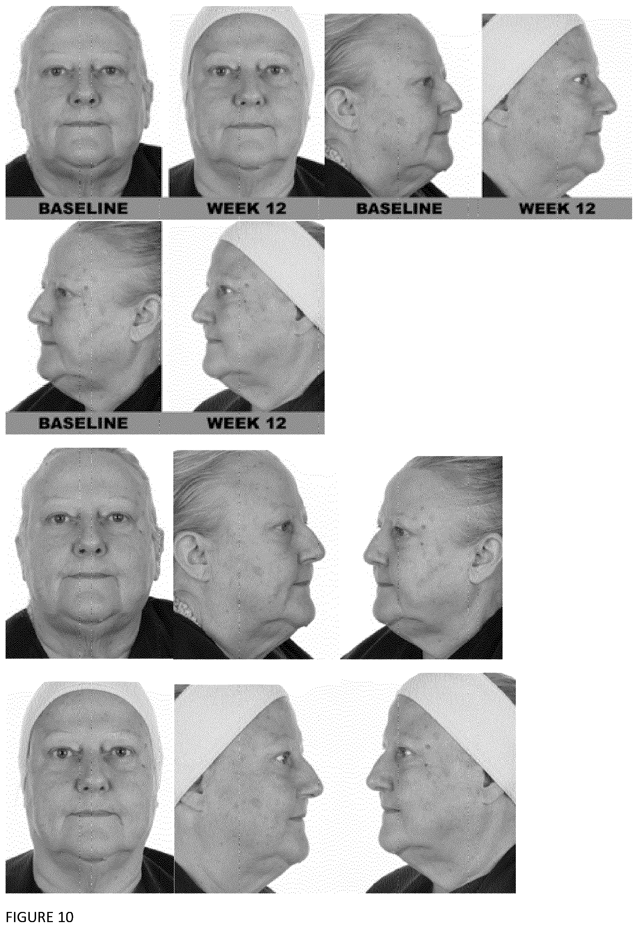

Figure 1 to 10 show the high-resolution clinical photographs of 10 subject's faces taken prior to application of the test article (Baseline - Day 0) and post treatment at study week 12. These images are at each timepoint: Frontal, left and right profile.

### Detailed Description of the Invention

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. Furthermore, there is no intention to be bound by any expressed or implied theory presented in the preceding technical field, background, brief summary or the following detailed description. Specific dimensions and other physical characteristics relating to the embodiments disclosed herein are therefore not to be considered as limiting, unless the claims expressly state otherwise.

The present invention now will be described hereinafter with reference to the accompanying examples, in which embodiments of the invention are shown. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. Thus, the invention contemplates that in some embodiments of the invention, any feature or combination of features set forth herein can be excluded or omitted. In addition, numerous variations and additions to the various embodiments suggested herein will be apparent to those skilled in the art in light of the instant disclosure, which do not depart from the instant invention. Hence, the following descriptions are intended to illustrate some particular embodiments of the invention, and not to exhaustively specify all permutations, combinations and variations thereof.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

Unless the context indicates otherwise, it is specifically intended that the various features of the invention described herein can be used in any combination. Moreover, the present invention also contemplates that in some embodiments of the invention, any feature or combination of features set forth herein can be excluded or omitted. To illustrate, if the specification states that a composition comprises components A, B and C, it is specifically intended that any of A, B or C, or a combination thereof, can be omitted and disclaimed singularly or in any combination.

Also as used herein, "and/or" refers to and encompasses any and all possible combinations of one or more of the associated listed items, as well as the lack of combinations when interpreted in the alternative ("or").

As used herein, the term "bacterium" or "probiotic bacterium" can refer to a single bacterial cell or a group of bacterial cells, and can be understood as and used interchangeably with the terms "bacterial strain," "bacterial species," and/or "bacterial population" as relevant to the context.

As used herein, the term "nutraceutical composition" can be understood as and used interchangeably with the terms "food supplement", "nutritional supplement" and/or "dietary supplement".

As used herein, the term "probiotic bacterium" further refers to a bacterium (e.g., a bacterium, bacterial strain, bacterial species, and/or bacterial population) whose presence may have beneficial effects on the health of a host (e.g, a subject, e.g, a mammalian subject e.g, a human subject). Non-pathogenic, commensal bacteria of the intestinal microbiota (also referred to as a "microbiome") may be probiotic bacteria. The term "probiotics" may also be used to refer to any composition that may contain live probiotic bacteria and/or bacterially derived compounds. Probiotics may be delivered in any format.

Without enough cellular energy, local facial skin stem cells are not able to proliferate for skin regeneration. After activation of local facial skin stem cells, the cells need energy, wherein the powerhouse (Mitochondria) of a cell provides the require energy to the cell, however, the powerhouse (Mitochondria) of facial skin cells becomes less efficient as we age.

One of the main causes of facial aging is a decline in mitochondrial function of facial skin cells which is directly linked to skin ageing phenotypes like wrinkle formation, uneven pigmentation, etc. Human facial skin cells protect themselves from this decline in mitochondrial function by stimulating the selective recycling of old or damaged mitochondria into new, healthy ones. This process is known as mitophagy. It's a natural process inside human cells to clean up defective and unhealthy mitochondria. Mitophagy becomes less efficient as we age. An outstanding discovery in 2016 and published in 2019 by author "Pénélope A. Andreux" et al. discloses the mitophagy activator urolithin A, which is safe and induces a molecular signature of improved mitochondrial and cellular health in humans, thus it is proved that Urolithin A is a mitophagy activator which create new, healthy mitochondria from old or damaged mitochondria of facial skin cells. Urolithin A is the only known compound that re-establishes cells' ability to recycle defective mitochondria.

Further it is discovered in a recent report of human clinical trials that some of human friendly intestinal microbes happily eat Punicalagins, Ellagitannin, Punicic acid, Ellagitannis and release Urolithin A.

Due to modern lifestyle and food habit, people lack enough such friendly microbes which can convert the Punicalagins, Ellagitannin, Punicic acid into Urolithin A. It is essential to increase the number of such microbes which can efficiently create Urolithin A within the body of human. According to a study only 30% of people now a days have such friendly microbes in their intestine, but still they do not have these friendly microbes sufficient in number in their intestine, further the food habit and adulterated foods decreases these friendly microbes presence in human body.

According to an embodiment of the present invention, there is a need to produce a food supplement or nutraceutical composition that not only improve natural production of Urolithin A within human body but also increases the presence of friendly microbes that can induce natural production of Urolithin A. Further the nutritional or food supplement probiotic composition of the present invention comprises pomegranate's Punicalagins, Ellagitannin, Punicic acid as a food for these friendly microbes which facilitate to create an ambient environment for the friendly microbes to live and multiply in human body while producing Urolithin A in good quantity and in natural way to facilitate and speed up mitophagy in human cells which is safe and induces a molecular signature of improved mitochondrial and cellular health in humans to create new, healthy mitochondria from old or damaged mitochondria in human skin cells and more specifically in facial skin cells. Thereby energizing skin cells to regenerate within few weeks to provide a distinguished glowing and rejuvenated glowing face.

Further according to another embodiment of the present invention, the naturally produced Urolithin A in human body by these friendly microbes in presence of Punicalagins and/or Ellagitannin and/or Punicic acid or a combination thereof is essential to pass the naturally produced Urolithin A to get absorbed in human intestine and transport to facial region in presence of several other ingredients as disclosed below to boost the absorption and transport of the ingredients to facial region for optimal facial skin cells regeneration and beautification.

According to another embodiment of the present invention the food supplement or nutraceutical composition comprises several selected vitamins, minerals, selected plant extracts, proteins etc in predetermined ratio to act as a skin care nutraceutical composition, wherein the skin care nutraceutical composition acts as a skin progenitor, thereby facilitating to provide a distinguished glowing and rejuvenated glowing skin and more specifically glowing facial skin.

According to another exemplary embodiment of the present invention the skin care nutraceutical composition comprises Organic Inulin, premix of probiotics, L-Citrulline, pomegranate extracts, pine bark extract (very soluble grade 98%), organic beet root extract, L.Arginine, organic watermelon rind extract, vitamin K2, and vitamin D3

According to another exemplary embodiment of the present invention the skin care nutraceutical composition comprises organic inulin, premix of probiotics, L-Citrulline, pomegranate extracts, pine bark extract (very soluble grade 98%), organic beet root extract, L-Arginine, organic watermelon rind extract, vitamin K2, vitamin D3, proferrin or lactoferrin, vitamin B3, vitamin B5 and vitamin B6.

According to another exemplary embodiment of the present invention the skin care nutraceutical composition comprises organic inulin, oligofructose, premix of probiotics, L-Citrulline, pomegranate extracts, pine bark extract (very soluble grade 98%), proferrin or lactoferrin, organic beet root extract, ashwagandha extract, vitamin C, L-Arginine, organic watermelon rind extract, vitamin B3, ZINC Bisglycinate, vitamin B6, vitamin B5, vitamin B9, vitamin K2, vitamin D3, L-Selenomethionine, and a flavor Silarom YUZU 1201304908.

According to another preferred embodiment of the present invention, the skin care nutraceutical composition comprises inulin and oligofructose are added in a 2:1 ratio in a predetermined range of 34.4% to 55% w/w of the total weight of the skin care nutraceutical composition.

According to another exemplary embodiment of the present invention premix of probiotics of the skin care nutraceutical composition comprises probiotics selected from Lactobacillus acidophilus, Lactobacillus delbrueckii subsp. Bulgaricus, Streptococcus thermophilus, Lactococcus lactis ssp. Cremoris, Lactobacillus helveticus and a combination thereof.

According to another exemplary embodiment of the present invention premix of probiotics of the skin care nutraceutical composition comprises probiotics selected from Lactobacillus acidophilus, Lactobacillus delbrueckii subsp. Bulgaricus, Streptococcus thermophilus, Lactococcus lactis ssp. Cremoris, Lactobacillus helveticus and a combination thereof in a predetermined range of 13.76% to 30% w/w of the total weight of the skin care nutraceutical composition. The skin care nutraceutical composition of the present invention comprises complete gut health blend of above probiotic strains, 20-30 billion CFUs (colony forming units) and Inuline, a beneficial prebiotic, works in perfect harmony to support vibrant, luminous skin from within our body.

Preferably, said probiotics are a combination of Lactobacillus acidophillus, Lactobacillus bulgaricus, Streptococcus thermophilus, Lactococcus lactis and Lactobacillus helveticus.

In a preferred embodiment, the said probiotics of the nutraceutical composition of the present invention are coated with an enteric coating. More preferably, this enteric coating comprises an internal protection against gastric and bile in the digestive tract which protects from degradation the skin care nutraceutical composition according to the invention when it enters into the stomach. This internal protection allows to protect the probiotic bacteria throughout ingestion, to allow them to reach the intestine alive and in good condition to colonize and proliferate. This internal protection can be combined with an external protection against moisture, heat, production, and transportation stresses. Furthermore, this coating layer increases stability during manufacture and shelf life of product.

Preferably, the probiotic bacterium which compound the premix (that is contained in the composition of the invention) are:
- Superior coated Lactobacillus acidophilus CBT LA1LAB2PRO^{™} which compound a viable cell count greater than 1.0 × 10¹¹ CFU/g and which compound the premix at a ratio up to 50.0%, preferably between 20% and 30%.
- Superior coated Lactobacillus bulgaricus CBT LG1LAB2PRO^{™} which compound a viable cell count greater than 1.0 × 10¹¹ CFU/g and which compound the premix at a ratio up to 0.5%, preferably between 0,2% and 0,4%.
- Superior coated Streptococcus thermophilus CBT ST3LAB2PRO^{™} which compound a viable cell count greater than 1.0 × 10¹¹ CFU/g and which compound the premix at a ratio up to 20%, preferably between 12% and 20%.
- Superior coated Lactococcus lactis CBT SL6LAB2PRO^{™} which compound a viable cell count greater than 1.0 × 10¹¹ CFU/g and which compound the premix at a ratio up to 21%, preferably between 15% and 21%.
- Superior coated Lactobacillus helveticus CBT LH5LAB2PRO^{™} which compound a viable cell count greater than 1.0 × 10¹¹ CFU/g and which compound the premix at a ratio up to 10%, preferably between 4% and 8%.

According to another exemplary embodiment of the present invention, the probiotic bacteria which compound the premix (that is contained in the composition of the invention) provides 25 billion CFU in 400 mg, and is composed of:
- Lactobacillus acidophilus - 200mg- 20 billion CFU
- Lactobacillus d. bulgaricus - 2 mg - 220 million CFU
- Streptococcus thermophilus - 78 mg - 7.8 billion CFU
- Lactococcus lactis - 80 mg - 80 mg - 8 billion CFU
- Lactobacillus helveticus - 40 mg - 4 billion CFU

According to another exemplary embodiment of the present invention pomegranate extracts of the skin care nutraceutical composition comprises Punicalagins, wherein probiotics in the intestine of human eat Punicalagins and produce Urolithin A, collagen and thousands of cellular and molecular factors for glow and beautiful skin. It activates the telomerase enzyme in optimal level. It helps to boost production of hyaluronic acid and collagen.

According to another exemplary embodiment of the present invention pomegranate extracts of the skin care nutraceutical composition comprises Ellagitannin, wherein probiotics in the intestine of human consume Ellagitannin and produce Urolithin A, collagen and thousands of cellular and molecular factors for glow and beautiful skin and activates the telomerase enzyme in optimal level. It helps to boost production of hyaluronic acid and collagen.

According to another exemplary embodiment of the present invention pomegranate extracts of the skin care nutraceutical composition comprises Punicic acid, wherein probiotics in the intestine of human eat Punicic acid and produce Urolithin A, collagen and thousands of cellular and molecular factors for glow and beautiful skin and activate the telomerase enzyme in optimal level. It helps to boost the production of hyaluronic acid and collagen. Antioxidant-rich Pomegranate extracts are loaded with natural compounds that help reduce cell damage caused by free radicals and defend against other environmental aggressors.

In a preferred embodiment the pomegranate extracts comprises in a predetermined range of 8.60% to 20% w/w of the total weight of the skin care nutraceutical composition.

According to another exemplary embodiment of the present invention the Lactoferrin of the skin care nutraceutical composition facilitate Urolithin A, hyaluronic acid and collagen to reach to the skin cells and at least to facial skin cells. Most of the existing market vitamins or food supplements are not beneficial because those are not able reach to target cells like skin cells and cause side effects in intestine. Further Lactoferrin a multitasking natural protein, effectively regulates the body's iron levels, promotes the growth of "good" bacteria for optimal gut health and facilitate to secrete collagen and more specifically collagen 17 in facial skin cell stem cells, stimulate Nitric oxide secretion on skin cells so as to improve oxygen and nutrient delivery to every cell in the body, including the cells of the facial skin.

In an alternate embodiment Proferrin can be interchangeably used in the place of Lactoferrin in the skin care nutraceutical composition. Heme iron polypeptide (HIP) is a new generation oral iron which uses the heme porphyrin ring to supply iron to sites of absorption in the intestinal lumen, thereby Proferrin being the Heme iron polypeptide (HIP) that is poorly soluble at the low gastric pH in our stomach, thus the availability of heme is unaffected by gastric secretion, as simply stomach enzymes can't digest it. Hence it can be used as food for the probiotics in the intestine and allows better absorption of the skin care nutraceutical composition of the present invention and its byproducts prepared in the intestine, thereby quickly and easily get transferred to skin cells for skin energizing and regeneration.

In a preferred embodiment the Lactoferrin or Proferrin or combination thereof comprises in a predetermined range of 3.44% to 7% w/w of the total weight of the skin care nutraceutical composition.

According to another exemplary embodiment of the present invention the Ashwagandha extract of the skin care nutraceutical composition facilitate collagen production; it contains high levels of antioxidants to protect skin from free radicals, infections or acne. Further Ashwagandha stimulates DHEA (an important precursor to estrogen and testosterone) to encourage the production of natural skin oils, improves blood flow and circulation to the skin for a fresh, fair and firm complexion. In a preferred embodiment the Ashwagandha extract comprises in a predetermined range of 3.44% to 15% w/w of the total weight of the skin care nutraceutical composition.

According to another exemplary embodiment of the present invention of the skin care nutraceutical composition further comprises oligofructose. The Oligofructose is a subgroup of the Inulin present in the composition, oligofructose is a preferred food for microbes (probiotics) in the initial part of the intestine, whereas the Inulin is the preferred food for the microbes (probiotics) at the remaining part (middle part to last part) of the intestine. The oligofructose facilitate to stimulate the proliferation of bifidobacteria who lives in first part of the intestine. The combined approach of oligofructose and inulin is like a complete fertilizer to multiply all friendly microbes (probiotics) in our intestine and suppressing potential pathogenic organisms in the intestine. In the preferred composition of the present invention Inulin and oligofructose are added in a 2:1 ratio in a predetermined range of 34.4% to 55% w/w of the total weight of the skin care nutraceutical composition. Thus, the skin care nutraceutical composition of the present invention facilitates to provide double actions to supply friendly probiotics and facilitated to provide a suitable environment to grow such friendly probiotics inside human intestine in an efficient manner, so as to facilitate skin regeneration factors for skin regeneration to make our skin glow naturally and from inside our body metabolism.

In an exemplary embodiment the skin care nutraceutical composition of the present invention supply 10 to 30 billions of friendly microbes as a premix of probiotics, in a preferred embodiment about 20 billions microbes into our body as a dietary supplement, wherein these microbes are supplied with food supplements for them such as inulin and oligofructose to eat at different stages and different parts of our intestine thereby facilitating quick multiply of the number of friendly microbes inside the intestine. All friendly microbes of complete intestine are the factory and secret of skin glow beauty. Taken together, ample number and the whole kingdom of all friendly intestinal microbes efficiently separate thousands of known and unknown skin regeneration factors for skin regeneration to make our skin glow naturally and from inside. In a preferred embodiment the premix of probiotics comprises in a predetermined range of 13.76% to 30% w/w of the total weight of the skin care nutraceutical composition.

According to another preferred embodiment of the present invention, the skin care nutraceutical composition comprises Proferrin or Lactoferrin in a predetermined range of 3.44% to 7% w/w of the total weight of the skin care nutraceutical composition.

According to another preferred embodiment of the present invention, the skin care nutraceutical composition comprises L-Citrulline in a predetermined range of 17.20% to 25% w/w of the total weight of the skin care nutraceutical composition. L-Citrulline stimulates the Nitric oxide (NO) and blood flow to skin cells thereby facilitate skin regeneration, hydration and skin glow.

According to another preferred embodiment of the present invention, the skin care nutraceutical composition comprises L-Arginine in a predetermined range of 1.72% to 3% w/w of the total weight of the skin care nutraceutical composition.

Both L-Arginine and L-Citrulline facilitate to stimulate and increase collagen production and synthesis, for strong, smooth energized skin.

High loss of nitric oxide production is one of the earliest events in the facial skin aging process and is responsible for the signs of aging including poor complexion, appearance of fine lines and wrinkles. Thus, human aged skin needs natural production of nitric oxide under skin to erase the all fine lines and wrinkles of face and make strong, youthful, elastic skin brighter, younger and hydrated. Watermelon rind extract and beet root extract can produce the natural nitric oxide in natural way of production under skin especially facial region. Beet root extract carry oxygen and iron and provides enough energy for skin cell regeneration.

Watermelon rind extract stimulates the secretion of nitric oxide of L-arginine and L-citrulline and helps in absorption of nutrients in the intestine. Further Watermelon rind extract reduces the stress proteins (heat sock protein and all stress proteins) in stomach, the mid intestine heat shock protein 27 (HSP27), and the colon heat shock protein 70 (HSP70). Further Watermelon rind extract increase the cellular (skin cells and blood circulation) level of superoxide dismutase (SOD). It is more powerful than antioxidant vitamin as it activates natural production of antioxidant inside human body including catalase and glutathione peroxidase.

According to another preferred embodiment of the present invention, the skin care nutraceutical composition comprises organic watermelon rind extract in a predetermined range of 0.69% to 10% w/w of the total weight of the skin care nutraceutical composition.

According to another preferred embodiment of the present invention, the skin care nutraceutical composition comprises organic beet root extract in a predetermined range of 3.44% to 10% w/w of the total weight of the skin care nutraceutical composition.

According to another preferred embodiment of the present invention, the skin care nutraceutical composition comprises Pine bark extract, wherein Pine bark extract has a significant impact on improving nitric oxide (NO) levels in the body thereby responsible for vasodilatation and improving blood flow, protect skin from sun damage, repair the damaged or injured DNA of skin cells, contributes to skin hydration, reduces pigmentation, and facilitate Telomerase activation. In an aspect the Pine bark extract (OPC DE PIN) of the composition uses a very soluble grade 98%, in a predetermined range of 2.41% to 6% w/w of the total weight of the skin care nutraceutical composition.

According to another preferred embodiment of the present invention, the skin care nutraceutical composition comprises vitamin C in a predetermined range of 4.13% to 15% w/w of the total weight of the skin care nutraceutical composition, wherein vitamin C is antioxidant-rich thus effectively evens out skin tone and hyper-pigmentation while boosting overall radiance.

According to another preferred embodiment of the present invention, the skin care nutraceutical composition comprises vitamin B3 in a predetermined range of 0.55 to 1% w/w of the total weight of the skin care nutraceutical composition, wherein vitamin B3 is essential to keep the skin well-hydrated and supple.

According to another preferred embodiment of the present invention, the skin care nutraceutical composition comprises ZINC (Bisglycinate or glycinate) in a predetermined range of 1.72% to 5% w/w of the total weight of the skin care nutraceutical composition. Anti-inflammatory Zinc supports skin renewal while addressing scars and wound healing, along with other skin concerns including rosacea to eczema.

According to another preferred embodiment of the present invention, the skin care nutraceutical composition comprises Vitamin B6 in a predetermined range of 0.05% to 1% w/w of the total weight of the skin care nutraceutical composition.

According to another preferred embodiment of the present invention, the skin care nutraceutical composition comprises Vitamin B5 in a predetermined range of 0.23% to 1% w/w of the total weight of the skin care nutraceutical composition, wherein Vitamin B5 and Vitamin B6 combat visible signs of aging, uneven skin texture, and acne.

According to another preferred embodiment of the present invention, the skin care nutraceutical composition comprises vitamin B9 in a predetermined range of 0.01% to 0.05% w/w of the total weight of the skin care nutraceutical composition, wherein Vitamin B9 increases the production of new skin cells while helping to reverse the negative effects of solar exposure.

According to another preferred embodiment of the present invention, the skin care nutraceutical composition comprises vitamin K2 in a predetermined range of 0.28% to 2% w/w of the total weight of the skin care nutraceutical composition, wherein vitamin K2 facilitate proper organization of synthesized collagens in facial skin cells thereby boosts skin elasticity to prevent wrinkles.

According to another preferred embodiment of the present invention, the skin care nutraceutical composition comprises vitamin D3 in a predetermined range of 0.10% to 0.45% w/w of the total weight of the skin care nutraceutical composition, wherein vitamin D3, enhances the skin's immune system and helps destroy free radicals that can trigger premature aging.

According to another preferred embodiment of the present invention, the skin care nutraceutical composition comprises L-Selenomethionine provides serious skin protection from UV damage by increasing the levels of selenium-dependent antioxidant proteins prior to UV exposure, wherein the L-Selenomethionine is added in the composition of the present invention in a predetermined range of 0.38% to 1% w/w of the total weight of the skin care nutraceutical composition.

According to another preferred embodiment of the present invention, the skin care nutraceutical composition comprises the flavor Silarom YUZU 1201304908 in a predetermined range of 3.44% to 5% w/w of the total weight of the skin care nutraceutical composition.

According to another exemplary embodiment of the present invention the skin care nutraceutical composition additionally comprises predetermined quantity of Moringa leave extract, wherein the Moringa leave extract is a natural source of vitamin E thereby facilitate to stimulate collagen production and reduces facial inflammation. Thus, Moringa leave extract facilitate to act as one of the best sources for vitamins and minerals in natural form which is required for skin regeneration. Stimulate collagen production and glutathione enzyme level and repair the damages DNA. Moringa leave extract enhances the human skin facial revitalization. It is a strong antioxidant activity by scavenging free radicals. It contains a high content of phenolic compounds, flavonoids. Moringa has many nutrients healthy for your skin including vitamin A, which builds collagen - the vital component that your skin is made of.

According to another exemplary embodiment of the present invention, the nutraceutical composition according to the invention comprises Moringa extract in a predetermined range of 8 % to 12 % w/w.

According to another exemplary embodiment of the present invention the skin care nutraceutical composition additionally comprises predetermined quantity of Bearberry Extract, wherein Bearberry extract inhibit facial pigmentation.

Bearberry extract is a botanical skin lightener with high concentration of arbutin (C12H16O7). It prevents melanin formation. Melanocyte produce brown spots which contain enzymes called tyrosinase. Such enzyme can activate anytime whenever they come in contact with UV light and develop freckles and sunspots after long term exposure of sun. In this case, the active ingredient of Bearberry extract comes in and block tyrosinase and keeps dark spots at bay. It acts as tyrosinase inhibitors. It reduces degree of skin darkening after sun exposure by blocking the production of tyrosinase. It also reduces skin pigmentation by diminishing melanin content in melanocytes.

According to another exemplary embodiment of the present invention, the nutraceutical composition according to the invention comprises bearberry extract in a predetermined range of 10 % to 15% w/w.

According to another exemplary embodiment of the present invention the skin care nutraceutical composition additionally comprises predetermined quantity of Curcuma extract.

Curcuma extract has anti-inflammatory and antioxidant properties. Curcuma extract induces endogenous cellular defence mechanisms against oxidative stress. As oxidative stress is one of the parameters which can lead to skin ageing, it is important to increase the components of the body's inherent antioxidative system. Curcuma extract is for the improvement of skin radiance and evenness of skin tone and also reduces the appearance of wrinkles in face.

According to another exemplary embodiment of the present invention, the nutraceutical composition according to the invention comprises curcuma extract in a predetermined range of 3 % to 5% w/w.

According to another exemplary embodiment of the present invention the skin care nutraceutical composition additionally comprises predetermined quantity of Root extract of Picrorhiza kurroa Royle ex Benth, wherein the Picrorhiza kurroa Royle ex Benth is main source of Apocyin (4'-hydroxy-3'-methoxyacetophenone or acetovanillone) that facilitate to repair the damaged DNA of human facial skin cells. Apocyin is one of main master key ingredients of this beauty capsule. The Lactoferrin present in the composition actively help the apocyin to reach facial skin cell via human intestine. Apocyin (4'-hydroxy-3'-methoxyacetophenone or acetovanillone) is identified as the biologically active substance in the roots of Picrorhiza kurroa Royle ex Benth, a perennial plant growing in the alpine Himalaya. But Japanese group discovered another small molecule (in adition to Apocyin) which is also create the collagen 17 production. For 2 decades, apocylin used for as treatment of neurodegenerative diseases, now, it is proved about the outstanding role for skin regeneration.

According to another exemplary embodiment of the present invention, the nutraceutical composition according to the invention comprises Root extract of Picrorhiza kurroa Royle ex Benth in a predetermined range of 7 % to 8% w/w.

According to another exemplary embodiment of the present invention the skin care nutraceutical composition additionally comprises predetermined quantity of Choline, which helps to restore elasticity and suppleness to the damaged skin cells.

The human skin cell needs choline to synthesize phosphatidylcholine and sphingomyelin, two major phospholipids vital for cell membranes. It has key role to maintain the proper levels of B vitamins that help in the production of collagen and elastin in the skin. Choline significantly increases antioxidant properties which help to preserve skin elasticity and prevent free radical damage to skin cell. Choline is needed to produce acetylcholine, an important neurotransmitter nervous system system. Skin regeneration coordinates by nervous system. In general, liver produce choline endogenously but the amount that the body naturally synthesizes is not sufficient to meet human needs. As a result, humans must obtain some choline from the dietary supplement.

According to another exemplary embodiment of the present invention, the nutraceutical composition according to the invention comprises choline in a predetermined range of 7 % to 12 % w/w.

According to another exemplary embodiment of the present invention the skin care nutraceutical composition additionally comprises predetermined quantity of snow mushroom also known as Tremella fuciformis. Wherein the snow mushroom added in a desired quantity in the skin care nutraceutical composition facilitate to boost skin hydration. It is also known in the art that fungus like snow mushroom acts similar to that of hydration powerhouse hyaluronic acid by pulling moisture to the skin. Hyaluronic acid can hold up to 1,000 times its weight in water, so finding an all-natural match for its hydrating power is pretty major. And snow mushroom has good hydrating power and acts similar to hyaluronic acid to hydrate the skin. Thus, snow mushroom improves skin elasticity through intense hydration, while simultaneously working to reduce signs of inflammation and, thereby facilitating skin glow. Snow mushroom extract contain 18 kinds of amino acids and has 400 times the hydrating power of hyaluronic acid. It generates a natural hydration optimally on the skin, enabling it to develop elasticity and a fit appearance. It also inhibits melanin formation. Snow mushroom extract has high levels of polysaccharides, which helps it draw moisture deep into the skin. It increases the look of elasticity, nourish and protect the skin by creating a natural barrier and locking in moisture. Snow mushroom also contains polysaccharides, which are anti-inflammatory and increase the functionality of the skin barrier by preventing water loss.

According to another exemplary embodiment of the present invention, the nutraceutical composition according to the invention comprises snow mushroom in a predetermined range of 15 % to 20 % w/w.

According to another exemplary embodiment of the present invention the skin care nutraceutical composition 100% w/w comprises organic inulin and oligofructose in 2:1 ratio in a predetermined range of 34.4% to 55% w/w; premix of probiotics in a predetermined range of 13.76% to 30% w/w; L-Citrulline in a predetermined range of 17.20% to 25% w/w; pomegranate extracts in a predetermined range of 8.60% to 20% w/w; Pine bark extract of 98% soluble grade in a predetermined range of 2.41% to 6% w/w; organic beet root extract in a predetermined range of 3.44% to 10% w/w; L-Arginine in a predetermined range of 1.72% to 3% w/w; organic watermelon rind extract in a predetermined range of 0.69% to 10% w/w; vitamin K2 in a predetermined range of 0.28% to 2% w/w; and vitamin D3 in a predetermined range of 0.10% to 0.45% w/w.

According to another exemplary embodiment of the present invention the skin care nutraceutical composition 100% w/w comprises organic inulin and oligofructose in 2:1 ratio in a predetermined range of 34.4% to 55% w/w; premix of probiotics in a predetermined range of 13.76% to 30% w/w; L-Citrulline in a predetermined range of 17.20% to 25% w/w; pomegranate extracts in a predetermined range of 8.60% to 20% w/w, wherein the pomegranate extracts comprises Punicalagins, Ellagitannin, Punicic acid and a combination thereof; Pine bark extract of 98% soluble grade in a predetermined range of 2.41% to 6% w/w; organic beet root extract in a predetermined range of 3.44% to 10% w/w; L-Arginine in a predetermined range of 1.72% to 3% w/w; organic watermelon rind extract in a predetermined range of 0.69% to 10% w/w; vitamin K2 in a predetermined range of 0.28% to 2% w/w; vitamin D3 in a predetermined range of 0.10% to 0.45% w/w; Proferrin in a predetermined range of 3.44% to 7% w/w; vitamin B3 in a predetermined range of 0.55 to 1% w/w; vitamin B5 in a predetermined range of 0.23% to 1% w/w; and vitamin B6 in a predetermined range of 0.05% to 1% w/w.

According to another exemplary embodiment of the present invention the skin care nutraceutical composition 100% w/w comprises organic inulin and oligofructose in 2:1 ratio in a predetermined range of 34.4% to 55% w/w; premix of probiotics in a predetermined range of 13.76% to 30% w/w; L-Citrulline in a predetermined range of 17.20% to 25% w/w; pomegranate extracts in a predetermined range of 8.60% to 20% w/w wherein the pomegranate extracts comprises Punicalagins, Ellagitannin, Punicic acid and a combination thereof; Pine bark extract of 98% soluble grade in a predetermined range of 2.41% to 6% w/w; organic beet root extract in a predetermined range of 3.44% to 10% w/w; L-Arginine in a predetermined range of 1.72% to 3% w/w; organic watermelon rind extract in a predetermined range of 0.69% to 10% w/w; vitamin K2 in a predetermined range of 0.28% to 2% w/w; vitamin D3 in a predetermined range of 0.10% to 0.45% w/w; Lactoferrin in a predetermined range of 3.44% to 7% w/w; vitamin B3 in a predetermined range of 0.55 to 1% w/w; vitamin B5 in a predetermined range of 0.23% to 1% w/w; vitamin B6 in a predetermined range of 0.05% to 1% w/w; Ashwagandha extract in a predetermined range of 3.44% to 15% w/w; vitamin C in a predetermined range of 4.13% to 15% w/w; ZINC Bisglycinate in a predetermined range of 1.72% to 5% w/w; vitamin B9 in a predetermined range of 0.01% to 0.05% w/w; L-Selenomethionine in a predetermined range of 0.38% to 1% w/w; and a flavor Silarom YUZU 1201304908 in a predetermined range of 3.44% to 5% w/w.

In scar-free healing the protein tissue growth factor beta chain (TGF-β) plays a pivotal role as wound healing promoting factor. It causes the release of extracellular matrix (ECM) molecules by fibroblasts for increased production of ECM proteins such as collagen, fibronectin and proteoglycans. TGF-β is a multifunctional growth factor that affects a wide range of cellular functions such as cell proliferation, differentiation, migration and adhesion, crucially. It has three isoforms with different functions. While TGF-β1 and β2 promote scarring and fibrosis, TGF-β3 seems to inhibit scarring. This assumption was deduced from studies of wounds of mammalian fetuses, which showed scar-free healing. The ratio of TGF-β1 was changed to TGF-β3 in adult, as opposed to wounds healed. Embryonic wounds with scar-free healing show an increased level of TGF-β3 and a degraded level of TGF-β1, as expected from the previous results. According to another embodiment of the present invention the probiotic skin care nutraceutical composition stimulates TGF-β3 on skin cells to generate scar free skin regeneration while reduce the secretion of TGF-β1 and TGF-β2, thereby facilitating skin regeneration at a faster rate to improve overall tone, texture, complexion and clarity of the skin.

The composition is a complete daily dose of ultra-potent skin superfoods created to nourish, hydrate, improve and illuminate from the inside out. The composition improves gut health, improves collagen synthesis, provides defense against environmental damage and inflammation, and helps in production of natural skin oils while promoting overall skin wellness. Combining bio-available vitamins, probiotics, prebiotics, adaptogens, antioxidants, anti-inflammatories, and amino. The skin ritual comes as an unflavored powder for optimal absorption, in a convenient single- serving size perfect for no-fuss preparation at home or on the go. Dissolve one sachet per day in a hot or cold beverage as part of our skincare regimen for an exceptionally vibrant, supple, resilient and healthy-looking glow.

In some embodiments, the probiotic skin care nutraceutical composition of the invention may be packaged in single or multiple doses in powder form as probiotic composition is a dosage form of a sachet, a tablet, a pellet, a hard capsule, a soft capsule, an emulsion, a powder, a dispersible powder, a lozenge, a troche, a chew, a gel, an aqueous or oily suspension, a spray, a granule, a suppository, a solution, a syrup, an elixir sachets, blister packs, and/or small or large containers containing multiple doses. Further the user can use one or two doses as per the requirement mixed in hot or cold beverages or any form of drinks like health drinks or can be mixed with other food items to be taken simultaneously as a dietary supplement for a prolonged use of at least 30 days. The composition of the present invention can be made available in any other form such as a beverage, a nutritional bar, a fermented dairy product, a non-fermented dairy product, or any combination thereof to the user in customized package as weekly dose or monthly dose or the like.

According to a second aspect, the invention relates to the use of a nutraceutical composition according to the invention for the skin care, preferably to improve and boost healthy skin function, improve the skin elasticity, increase the synthesis of collagen, reduce the appearance of uneven pigmentation and texture, hydrate the skin, and/or prevent the appearance of wrinkles and fine lines on skin.

According to a third aspect, the invention relates to a nutraceutical composition according to the invention for use in reducing inflammation.

According to a fourth aspect, the invention relates to a cosmetic care method comprising the oral intake of a nutraceutical composition for skin care according to the invention.

The invention will now be described with reference to the following examples. It should be appreciated that these examples are not intended to limit the scope of the claims to the invention, but are rather intended to be exemplary of certain embodiments. Any variations in the exemplified methods that occur to the skilled artisan are intended to fall within the scope of the invention.

### EXAMPLE 1

**Table: 1 (Exemplary probiotic skin care nutraceutical composition 1)**

| **Probiotic skin care nutraceutical composition** | **mg / Stick** |
|---|---|
| Organic Inulin and oligofructose in 2:1 ratio (ATW 90%) | 1000.00 |
| Premix of 5 Probiotics 20-25 billion | 400.00 |
| L-Citrulline | 500.00 |
| Pomegranate ES 40% punicosides | 250.00 |
| Pine bark extract (OPC DE PIN) in 98% soluble grade | 70.00 |
| Organic Beet extract 4: 1 | 100.00 |
| L-ARGININE | 50.00 |
| Organic Watermelon rind extract | 20.00 |
| Vitamin K2 100% VNR | 8.25 |
| Vitamin D3 (0.25%) 100% VNR | 3.00 |
| **Total** | **2401.25 mg** |

### EXAMPLE 2

**Table: 2 (Exemplary probiotic skin care nutraceutical composition 2)**

| **Probiotic skin care nutraceutical composition** | **mg / Stick** |
|---|---|
| Organic Inulin and oligofructose in 2:1 ratio (ATW 90%) | 1000.00 |
| Premix of 5 Probiotics 20-25 billion | 400.00 |
| L-Citrulline | 500.00 |
| Pomegranate ES 40% punicosides | 250.00 |
| Pine bark extract (OPC DE PIN) in 98% soluble grade | 70.00 |
| Proferrin or Lactoferrin | 100.00 |
| Organic Beet extract 4: 1 | 100.00 |
| Ashwagandha extract 2.5% withanolides | 100.00 |
| Vitamin C 100% VNR 97.5% coated | 120.00 |
| L-ARGININE | 50.00 |
| Organic Watermelon rind wxtract | 20.00 |
| Vitamin B3 NIACIN 100% VNR | 16.00 |
| ZINC Bisglycinate (20% Zn) 100% VNR | 50.00 |
| Vitamin B6 Pyridoxine 5'- phosphate 100% VNR | 1.54 |
| Vitamin B5 100% VNR Calcium D-Pantothenate | 6.60 |
| Vitamin B9 Folic acid 100% VNR | 0.22 |
| Vitamin K2 100% VNR | 8.25 |
| L-Selenomethionine (0.5% Se) 100% VNR | 11.00 |
| Vitamin D3 (0.25%) 100% VNR | 3.00 |
| Silarom YUZU 1201304908 | 100.00 |
| **Total** | **2906.61 mg** |

The above tables 1 and 2 show exemplary probiotic skin care nutraceutical compositions in a sachet or stick form, which can be mixed with any drinks or food or taken independently as a nutraceutical supplement. The approx 2 to 5 gram sachet is a convenient single serving size that is perfect for no-fuss preparation at home or on the go. Dissolve one sachet in a hot or cold beverage or in water as part of skin care nutritional supplement. The probiotic composition sachet taken regularly once or twice a day for few weeks improves gut health, improves collagen synthesis, promote mitophagy, stimulate TGF-β3, provides defense against environmental damage and inflammation, and helps in production of natural skin oils while promoting overall skin wellness.

These and other advantages of the present invention will be apparent to those skilled in the art from the above specification. Accordingly, it will be recognized by those skilled in the art that changes or modifications may be made to the above-described embodiments without departing from the broad inventive concepts of the invention. Specific dimensions of any particular embodiment are described for illustration purposes only.

### Example 3: 12 Week User Trial Results: skin supplements of the invention alone.

A clinical study in 105 healthy volunteers to assess the efficacy of the nutraceutical composition according to the invention via clinical, visual, and tacticle assessments and self-perception questionnaires.

### Study design: Single-blind, clinical study.

Study Method: Day - 3 (Wash out period) Subjects will attend the study facility where informed consent will be obtained, and eligibility will be verified. Once accepted, subjects will be given a bland bar of soap to use for the next 3 days. Subjects will NOT use any lotions, creams, or moisturization products during this period.

Day 0 (Baseline) Subjects will return to the testing facility following the 3 day wash out period, they will be queried for any changes to their health or medication since their last visit. Following this, 35 subjects will be randomly selected for clinical, visual, and tactile assessments. They will undergo these assessments at baseline and at weeks 2, 4 and 12. All subjects will be given an information sheet along with a subject diary to complete each time they use the product and enough of the test article to last the duration of the study. Subjects that were not selected for clinical, visual, and tactile assessments will be given a time slot for their next visit and allowed to leave. The selected subjects will then undergo clinical, visual, and tactile assessments as their baseline readings. From the selected 35 subjects, a further 10 subjects will be selected to have before and after photos taken of their face. The selected subjects will sign a photograph release form and have the photos taken at baseline. Subjects will then be given a time slot for their next visit and be allowed to leave.

Day 14 (Visit 3) - Subjects will return to the study facility following 14 days of home use. All subjects will be queried for any changes to their health or medication since their last visit, subject dairies will also be checked for compliance. All subjects will then be given a self-perception questionnaire to complete on how they found using the product over the 14 Day period. Once completed the subjects that were not selected for clinical, visual, and tactile assessments will be given a time slot for their next visit and be allowed to leave.

The 35 selected subjects will undergo the same clinical, visual and tactile assessments as the baseline visit before being given a time slot for their next visit.

Day 28 (Visit 4) - Subjects will return to the study facility following 28 days of home use. All subjects will be queried for any changes to their health or medication since their last visit, subject dairies will also be checked for compliance. All subjects will then be given a self-perception questionnaire to complete on how they found using the product over the 28 Day period. Once completed the subjects that were not selected for clinical, visual, and tactile assessments will be given a time slot for their next visit and be allowed to leave. The 35 selected subjects will undergo the same clinical, visual, and tactile assessments as the baseline visit before being given a time slot for their next visit.

Day 84 (Visit 5) - Subjects will return to the study facility following 84 days of home use. All subjects will be queried for any changes to their health or medication since their last visit, subject dairies will also be checked for compliance. All subjects will then be given a self-perception questionnaire to complete on how they found using the product over the 84 Day period. Once completed the subjects that were not selected for clinical, visual, and tactile assessments will be compensated for their time and be allowed to leave. The 35 selected subjects will undergo the same clinical, visual, and tactile assessments as the baseline visit. The subjects that were not selected for photographs will be compensated for their time and be allowed to leave. The selected subjects for photographs will have their photographs taken and then be compensated for their time and allowed to leave.

### Duration of study: 12 weeks.

Number of subjects: Minimum 105 subjects to complete the study (35 of which will undergo clinical assessments and 10 subjects for photographs)

Types of subjects: Healthy male and female volunteers, between the ages of 18 and 65 (25% Asian, black, Hispanic) age: 35-65 with aging skin, dull, uneven tone/texture skin.

### SUBJECTS:

### INCLUSION CRITERIA

a. Healthy male and female volunteers, between the ages of 18 and 70 years.
b. Subject has aging skin.
c. Subject has dull skin.
d. Subject has uneven tone/texture skin.
e. Subject has signed a written Informed Consent.

### EXCLUSION CRITERIA

a. Subject is pregnant, nursing, or planning to become pregnant.
b. Subject is currently using concurrent medication likely to affect the response to the test article or confuse the results of the study including anti-depressants, and Botox/collagen fillers.
c. Heavy alcohol consumption in the opinion of the investigator.
d. A fever in the last 12 hours, prior to start of the study.
e. Significant past medical history of hepatic, cancerous, multiple sclerosis, high blood pressure, renal, Thrombosis/Phlebitis, cardiac, pulmonary, digestive, haematological, neurological, locomotor or psychiatric disease, which in the opinion of the Investigator would compromise the safety of the subject.
f. Insulin-dependent diabetes.
g. Concurrent medication likely to affect the response to the test article or confuse the results of the study including Botox/collagen fillers.
h. Subject is not currently participating, at PCR or other clinical testing facility, in a study consuming any supplements.
i. Pacemaker.
j. Photo Epilepsy for Light Therapy.

### PROHIBITIONS & RESTRICTION

a. Subject agrees to not use any anti-wrinkle products for the duration of the study.
b. Subject agrees to not use any firming/elasticity products for the duration of the study.
c. Subject agrees to not use any moisturization products for the duration of the study.
d. Subject agrees to only use (as instructed) the test article provided for the duration of the study.
e. Avoid Area: Metal pins/plates or silicone implants in face, open cuts and abrasions, skin and eye infections, severe sunburn, conjunctivitis, styes, and in-flare eczema/psoriasis on face.
f. Subject agrees to attend for all visits with clean face, free of makeup.

### INSTRUMENTAL ASSESSMENTS

a. PROFILOMETRY ASSESSMENTS

### Skin surface replicas and Profilometry analysis

A skin surface replica will be taken of the crow's foot area (lateral canthus lines) of either the right or left side of the face following procedures outlined below.

### Site identification and relocation

The following procedure will be used to identify replica location and facilitate relocation at subsequent visits.

Subjects will be positioned on their backs with their face to the side, looking over their shoulder. They will be asked to close their eyes and maintain a neutral expression. Test sites will be located using replica locating rings ensuring that each ring lies flat on the skin. The skin will not be stretched or pulled during ring placement. The ring will be placed in the centre of the crow's feet area with the tab directed towards the hairline. The foam and paper portions of each ring will be aligned. The transparency film will then be placed over the subject's face. Full locating ring, with centres, will then be placed onto the film exactly over the site which has been selected. Landmarks will then be traced onto the film using an indelible marker pen. The film will then be removed from the face and labelled to identify the subject. The film will then be stored in a cool, dry location until next use.

### Replica generation

The subject will be placed in an identical manner to that described above and landmarks on the transparency film will be lined up with the subject's facial features. A skin marker pen will then be used to make dots through the film onto the face of the subject to enable exact location of the test sites. The ring will then be positioned on the face and the replicas generated by filling the well in the centre of the ring with Silflo (JS Davis, Hart) material. Once the replica has set completely (approximately 5 minutes) it will be removed from the skin, allowed to dry skin side up for a few minutes, and will then be placed in a storage sleeve.

### Profilometric analysis

The following equipment and software will be used:

### Equipment

PC: IBM compatible Pentium III 500Mhz with 1 gb memory running under Windows XP Professional.

Video: SONY solid state B&W camera, 50mm lens/30mm extension, Coreco TCI Ultra frame grabber.

### Software: OPTIMAS v6.5, Microsoft EXCEL 2007, StatSoft STATISTICA 7.

Lighting: Collimated light source directed at a 25 angle from the plane of the replica. The replica will be placed in a holder that fixes the direction of the tab position of the replica so that the replica can be rotated to align the tab direction normal or parallel to the incident light direction.

### Tab Direction "Normal"

Unless specified otherwise, 2 sets of measurements will be produced for each replica: Normal (N) and Parallel (P). Interpretation of the results depends on the direction of the Tab relative to features of the skin surface.

The general background gradient of light intensity will be adjusted by applying a 1st order correction in the direction of the light propagation. The shadow texture produced by the oblique lighting of the negative replica will be analysed by the following methods:

Measuring the luminance along a set of 10 equal length parallel lines (passes) running across the replica parallel to the lighting direction. The variations in luminance will be treated as indicative of the roughness and analysed by traditional surface roughness statistics:

Rz- reports the average maximum difference in luminance value for five equal length segments in each of the 10 lines traversing the sample.

### b. CUTOMETER MPA 580

Measurements to study any changes in the viscoelastic properties of the skin by the test article will be performed using the Cutometer MPA 580 (Courage and Khazaka, Germany). The measuring principle is based on the suction method. Negative pressure is created in the device and the skin is drawn into the aperture of the probe. Inside the probe, the penetration depth is determined by a non-contact optical measuring system. This optical measuring system consists of a light source and a light receptor, as well as two prisms facing each other, which project the light from transmitter to receptor. The light intensity varies due to the penetration depth of the skin. The resistance of the skin to be sucked up by the negative pressure (firmness) and its ability to return into its original position (elasticity) are displayed as curves at the end of each measurement using Windows based software. The instrument will be used on the periorbital/crow's feet area of the face.

R0 and R1 values with be used to evaluate firmness and elasticity.

### c. CHROMAMETER ASSESSMENTS OF SKIN HYDRATION

Instrument assessments will be done with the Chromameter CR200 (Konica Minolta, Japan). The measuring head of the CR-200 uses diffuse illumination/0 viewing geometry. A pulsed xenon arc (PXA) lamp inside a mixing chamber provides diffuse, uniform lighting over the 8mm-diameter specimen area. Only the light reflected perpendicular to the specimen surface is collected by the optical-fiber cable for color analysis. The CIE L*a*b* color space will be utilized to quantify changes in skin color using the parameters, L*a*b*. Triplicate readings will be taken of the designated dark spot.

### d. CORNEOMETER ASSESSMENTS OF SKIN HYDRATION

Moisturisation measurements to study the humectant properties of the test article will be performed using the Corneometer CM825 (Courage and Khazaka, Germany). This instrument relies on the dielectric constant, a physical property of water, which is relatively high and as such will affect the capacitance of a capacitor. Any change in the dielectric constant due to skin moisture variations will alter the capacitance of the precision capacitor in the instrument. These variations are detected electronically and are converted into a value by the Corneometer. A 15-minute warm-up period will be allowed before using the Corneometer.

Three measurements will be made using the probe attachment of the Corneometer at each of the test sites (cheek area), between each assessment the probe attachment of the Corneometer will be pressed onto a dry tissue. The next assessment will not be performed until a value of 5 or less is displayed by the instrument. Subjects must have been in the controlled environment (at a temperature of 22 C 2 C and at a relative humidity of 45% 5%) for at least 30 minutes prior to any assessments being performed.

### e. TEWAMETER ASSESSMENTS OF TEWL (TRANS-EPIDERMAL WATER LOSS)

Transepidermal Water Loss (TEWL) measurements will be performed using the Tewameter TM300 (Courage and Khazaka, Germany). The measurement of the water evaporation, and therefore TEWL, is based on the diffusion principle in an open chamber. The density gradient is measured indirectly by two pairs of sensors in the probe attachment, one for temperature and the other for relative humidity. This density gradient is then analysed by a microprocessor in the instrument. A 15-minute warm-up period will be allowed before using the Tewameter.

### VISUAL ASSESSMENT

**Table: 3 - Criteria for visual assessments.**

| PCR Clinical Grading Scale | | | | | | | | | | | |
|---|---|---|---|---|---|---|---|---|---|---|---|
| Descriptors | Type of Grading | 0 | 1 | 2 | 3 | 4 | 5 | 6 | 7 | 8 | 9 |
| Global Fine lines | Visual | No fine lines | Mild fine lines | | | Moderate fine lines | | | Severe fine lines | | |
| Global Wrinkles | Visual | No wrinkles | Mild wrinkles | | | Moderate wrinkles | | | Severe wrinkles | | |
| Skin Sagging along jaw line | Visual | None (Best possible condition) | Mild | | | Moderate | | | Severe Worse Possible Condition | | |
| Skin Firmness | Tactile | Loose, lax feeling skin that moves freely upon manipulation | Mildly firm | | | Moderately firm | | | Firm, tight feeling that resists manipulation | | |
| Skin Elasticity | Tactile | Severe Worse Possible Condition | Moderate | | | Mild | | | None (Best possible condition) | | |
| Skin Tone Evenness | Visual | Uneven skin tone | Mildly even skin tone | | | Moderately even skin tone | | | Even skin tone | | |
| Skin Texture | Visual | Rough, hard uneven looking skin texture | Mildly smooth looking | | | Moderately smooth looking | | | Smooth, soft even looking skin texture | | |
| Skin Texture | Tactile | Rough, hard uneven skin texture | Mildly smooth | | | Moderately smooth | | | Smooth, soft even skin texture | | |
| Skin Radiance / Luminosity | Visual | Total dullness, matte appearance | Mild radiance | | | Moderate radiance | | | Total brightness, translucent appearance | | |
| Overall Skin Quality/ Healthy Appearance | Visual | Extremely healthy, vibrant, and radiant in appearance | Healthy appearance with minimal areas for improvement. | | | Healthy appearance with moderate areas for improvement. | | | Dull, lax, and unhealthy in appearance | | |
| Overall Hyperpigmentation/ skin discoloration (mottled pigmentation) | Visual | Very clear skin, no visible dark/ sunspots | Mostly clear skin, slightly visible dark/sunspots | | | Somewhat clear skin with some visible dark/ sunspots | | | No skin clarity/ extremely visible dark sunspots | | |
| Skin Blemishes | Visual | No visible redness | Minor redness | | | Moderate redness | | | Severe redness | | |

| | | | | | | | | | | | |
|---|---|---|---|---|---|---|---|---|---|---|---|
| *Skin Firmness (Suborbital/Crow's Feet by indentation) *Skin Elasticity (Suborbital/Crow's Feet by Skin pulling or pinch test/recoil | | | | | | | | | | | |

Subjects will be evaluated under standard lighting conditions and the same assessor will evaluate according to the scale, at each assessment point.

### SELF-PERCEPTION QUESTIONNAIRE (SPQ)

Subjects will complete a self-perception questionnaire (SPQ) at weeks 2, 4 and 12.

### CLINICAL PHOTOGRAPHY

High resolution clinical photographs of the subject's faces will be taken prior to application of the test article (Baseline - Day 0) and post treatment at study week 12. Three images will be taken at each timepoint: Frontal, left and right profile. Subjects will be instructed that their hair must be tidy and styled the same way at each visit. Subjects must have a neutral expression and each photo must be well/lit and exposed. All jewellery worn by the subjects must be removed prior to any images being taken.

### The following equipment and settings will be used to capture the images:

- Camera: Nikon D5500· Lens: Nikon 18-140 mm· All Images shot on camera raw at ISO 200 1/125th @ F20• Main light: 4X Soft box @135W• Fill light Speed Lite - Camera Mounted- Background light Speed Lite - remote

### Results:

### Self-perception questionnaire results:

**Table: 4 - User trial Results after 12 weeks)**

| **Product(s)** | **12 Week User Trial Results: Number of subjects: 105** |
|---|---|
| | **Type of subjects: Healthy male and female volunteers, between the ages of 18 and 65 with aging skin, dull, uneven tone/texture skin.** |
| **Skin Rejuvenating Complex** | **HYDRATION:** |
| | The skin supplement hydrated my skin: **93%** |
| | The skin supplement left my skin feeling moisturized: **95%** |
| | **FINE LINES + WRINKLES:** |
| | The skin supplement prevented wrinkles from appearing on my skin: **84%** |
| | The skin supplement reduced the appearance of fine lines + wrinkles: **91%** |
| | **SKIN FIRMNESS:** |
| | The skin supplement firmed my skin: **94%** |
| | The skin supplement increased my skin elasticity: 93 % |
| | **SHIN RADIANCE:** |
| | The skin supplement helped my skin appear more radiant/luminous: **94%** |
| | **SKIN TONE + TEXTURE:** |
| | The skin supplement reduced the appearance of uneven pigmentation: **92%** |
| | The skin supplement helped even out my skin texture: **95%** |
| | The skin supplement reduced skin irritation and redness: **94%** |
| | **SKIN IMPERFECTIONS + BLEMISHES:** |
| | The skin supplement reduced the appearance of skin imperfections: **96%** |
| | The skin supplement helped reduce excess shine on my skin: **97%** |
| | The skin supplement reduced the appearance of blemishes: **94%** |
| | The skin supplement helped prevent new blemishes: 95% |
| | The skin supplement helped improve the way my skin looks: **97%** |
| | **OVERALL SKIN HEALTH + WELLNESS:** |
| | The skin supplement helped with my overall skin health: **97%** |
| | After using this product, my skin looks and feels like it is renewed and restored: **94%** |
| | The skin supplement improved my digestion: **93%** |
| | The skin supplement helped me feel less bloated: **94%** |
| | The skin supplement helped me feel more at ease: 92% |
| | The skin supplement improved my mood/frame of mind: 90% |
| | The skin supplement helped reduce my stress levels: 91 % |
| | The skin supplement helped me feel less irritated: **94%** |
| | The skin supplement helped increase my energy level: **93%** |

### Clinical Trial Results:

**Table: 5 (clinical trial Results after 12 weeks)**

| **Product(s)** | **12 Week Clinical Trial Results: Number of subjects: 35 Type of subjects: Healthy male and female volunteers, between the ages of 18 and 65 with aging skin, dull, uneven tone/texture skin.** |
|---|---|
| **Skin Rejuvenating Complex** | **HYDRATION:** |
| | The skin supplement hydrated the skin by **160.47%** after 12 weeks |
| | The skin supplement combated dehydration by **25.07%** after 12 weeks |
| | **FINE LINES + WRINKLES:** |
| | The skin supplement visibly reduced fine lines by 60.51% after 12 weeks |
| | The skin supplement visibly reduced wrinkles by 52.01% after 12 weeks |
| | **SKIN FIRMNESS:** |
| | The skin supplement visibly improved skin firmness by 180.60% after 12 weeks |
| | The skin supplement visibly reduced skin sagging by 56.55% after 12 weeks |
| | The skin supplement visibly improved skin elasticity by 207.93% after 12 weeks |
| | **SKIN RADIANCE:** |
| | The skin supplement visibly improved skin radiance/luminosity by 204.64% after 12 weeks |
| | **SKIN TONE** + **TEXTURE:** |
| | The skin supplement visibly reduced hyperpigmentation by 37.90% after 12 weeks |
| | The skin supplement visibly improved even skin texture by 183.33% after 12 weeks |
| | The skin supplement improved the feel of my skin texture by 206.06% after 12 weeks |
| | The skin supplement visibly improved even skin tone by **185.48%** after 12 weeks |
| | **SKIN IMPERFECTIONS + BLEMISHES:** |
| | The skin supplement visibly reduced the look of skin imperfections by **51.05%** after 12 weeks |
| | The skin supplement visibly reduced the look of blemishes by 58.96% after 12 weeks |

### Clinical photography results

Figure 1 to 10 show the high-resolution clinical photographs of 10 subject's faces taken prior to application of the test article (Baseline - Day 0) and post treatment at study week 12. These photographs show the good efficacy of the nutraceutical composition according to the invention.

## Claims

1. A nutraceutical composition for skin care, comprising :
- Organic inulin and oligofructose in 2:1 ratio in a range of 34.4% to 55% w/w;
- Probiotics in a range of 13.76% to 30% w/w;
- L-Citrulline in a range of 17.20% to 25% w/w;
- Pomegranate extracts in a range of 8.60% to 20% w/w;
- Pine bark extract of 98% soluble grade in a range of 2.41% to 6% w/w;
- Organic beet root extract in a range of 3.44% to 10% w/w;
- L-Arginine in a range of 1.72% to 3% w/w;
- Organic watermelon rind extract in a range of 0.69% to 10% w/w;
- Vitamin K2 in a range of 0.28% to 2% w/w;
- Vitamin D3 in a range of 0.10% to 0.45% w/w.

2. The nutraceutical composition for skin care according to claim 1, wherein the probiotics are selected from Lactobacillus acidophilus, Lactobacillus delbrueckii subsp. Bulgaricus, Streptococcus thermophilus, Lactococcus lactis ssp. Cremoris, Lactobacillus helveticus and a combination thereof.

3. The nutraceutical composition for skin care according to any one of the preceding claims, wherein the pomegranate extracts comprise Punicalagins, Ellagitannin, Punicic acid or a combination thereof.

4. The nutraceutical composition for skin care according to any one of the preceding claims, wherein the composition further comprises Lactoferrin in a range of 3.44% to 7% w/w

5. The nutraceutical composition for skin care according to any one of the preceding claims, wherein the composition further comprises vitamin B3 in a range of 0.55 to 1% w/w.

6. The nutraceutical composition for skin care according to any one of the preceding claims, wherein the composition further comprises vitamin B5 in a range of 0.23% to 1% w/w.

7. The nutraceutical composition for skin care according to any one of the preceding claims, wherein the composition further comprises vitamin B6 in a range of 0.05% to 1% w/w.

8. The nutraceutical composition for skin care according to any one of the preceding claims, wherein the composition further comprises Ashwagandha extract in a range of 3.44% to 15% w/w.

9. The nutraceutical composition for skin care according to any one of the preceding claims, wherein the composition further comprises vitamin C in a range of 4.13% to 15% w/w.

10. The nutraceutical composition for skin care according to any one of the preceding claims, wherein the composition further comprises Zinc Bisglycinate in a range of 1.72% to 5% w/w.

11. The nutraceutical composition for skin care according to any one of the preceding claims, wherein the composition further comprises vitamin B9 in a range of 0.01% to 0.05% w/w.

12. The nutraceutical composition for skin care according to any one of the preceding claims, wherein the composition further comprises L-Selenomethionine in a range of 0.38% to 1% w/w.

13. The nutraceutical composition for skin care according to any one of the preceding claims, wherein the composition further comprises yuzu flavor in a range of 3.44% to 5% w/w.

14. The nutraceutical composition for skin care according to any one of the preceding claims, wherein the composition further comprises Moringa leave extract.

15. The nutraceutical composition for skin care according to any one of the preceding claims, wherein the composition further comprises Bearberry extract.

16. The nutraceutical composition for skin care according to any one of the preceding claims, wherein the composition further comprises Curcuma extract.

17. The nutraceutical composition for skin care according to any one of the preceding claims, wherein the composition further comprises Root extract of Picrorhiza kurroa Royle ex Benth.

18. The nutraceutical composition for skin care according to any one of the preceding claims, wherein the composition further comprises Choline.

19. The nutraceutical composition for skin care according to any one of the preceding claims, wherein the composition further comprises Snow mushroom.

20. The nutraceutical composition for skin care according to any one of the preceding claims, wherein the probiotics are coated with an enteric coating.

21. Non-therapeutic use of a nutraceutical composition according to any one of the preceding claims for the skin care, preferably to improve and boost healthy skin function, improve the skin elasticity, increase the synthesis of collagen, reduce the appearance of uneven pigmentation and texture, hydrate the skin, and/or prevent the appearance of wrinkles and fine lines on skin.

22. A nutraceutical composition according to any one of claims 1 to 20, for use in reducing inflammation.

23. A cosmetic care method comprising the oral intake of a nutraceutical composition for skin care according to any one of claims 1 to 20.

## Patentansprüche

1. Nutrazeutische Zusammensetzung für Hautpflege, umfassend:
- Organisches Inulin und Oligofructose in einem Verhältnis 2 : 1 in einem Bereich von 34,4 Gew.-% bis 55 Gew.-%;
- Probiotika in einem Bereich von 13,76 Gew.-% bis 30 Gew.-%;
- L-Citrullin in einem Bereich von 17,20 Gew.-% bis 25 Gew.-%;
- Granatapfelextrakte in einem Bereich von 8,60 Gew.-% bis 20 Gew.-%;
- Kiefernrindenextrakt mit einem Löslichkeitsgrad von 98 % in einem Bereich von 2,41 Gew.-% bis 6 Gew.-%;
- Bio-Rübenextrakt in einem Bereich von 3,44 Gew.-% bis 10 Gew.-%;
- L-Arginin in einem Bereich von 1,72 Gew.-% bis 3 Gew.-%;
- Bio-Wassermelonenschalenextrakt in einem Bereich von 0,69 Gew.-% bis 10 Gew.-%;
- Vitamin K2 in einem Bereich von 0,28 Gew.-% bis 2 Gew.-%;
- Vitamin D3 in einem Bereich von 0,10 Gew.-% bis 0,45 Gew.-%.

2. Nutrazeutische Zusammensetzung für Hautpflege nach Anspruch 1, wobei die Probiotika aus Lactobacillus acidophilus, Lactobacillus delbrueckii subsp. Bulgaricus, Streptococcus thermophilus, Lactococcus lactis ssp. Cremoris, Lactobacillus helveticus und einer Kombination davon ausgewählt sind.

3. Nutrazeutische Zusammensetzung für Hautpflege nach einem der vorstehenden Ansprüche, wobei die Granatapfelextrakte Punicalagine, Ellagitannin, Punicinsäure oder eine Kombination davon umfassen.

4. Nutrazeutische Zusammensetzung für Hautpflege nach einem der vorstehenden Ansprüche, wobei die Zusammensetzung ferner Lactoferrin in einem Bereich von 3,44 Gew.-% bis 7 Gew.-% umfasst.

5. Nutrazeutische Zusammensetzung für Hautpflege nach einem der vorstehenden Ansprüche, wobei die Zusammensetzung ferner Vitamin B3 in einem Bereich von 0,55 bis 1 Gew.-% umfasst.

6. Nutrazeutische Zusammensetzung für Hautpflege nach einem der vorstehenden Ansprüche, wobei die Zusammensetzung ferner Vitamin B5 in einem Bereich von 0,23 Gew.-% bis 1 Gew.-% umfasst.

7. Nutrazeutische Zusammensetzung für Hautpflege nach einem der vorstehenden Ansprüche, wobei die Zusammensetzung ferner Vitamin B6 in einem Bereich von 0,05 Gew.-% bis 1 Gew.-% umfasst.

8. Nutrazeutische Zusammensetzung für Hautpflege nach einem der vorstehenden Ansprüche, wobei die Zusammensetzung ferner Ashwagandhaextrakt in einem Bereich von 3,44 Gew.-% bis 15 Gew.-% umfasst.

9. Nutrazeutische Zusammensetzung für Hautpflege nach einem der vorstehenden Ansprüche, wobei die Zusammensetzung ferner Vitamin C in einem Bereich von 4,13 Gew.-% bis 15 Gew.-% umfasst.

10. Nutrazeutische Zusammensetzung für Hautpflege nach einem der vorstehenden Ansprüche, wobei die Zusammensetzung ferner Zinkbisglycinat in einem Bereich von 1,72 Gew.-% bis 5 Gew.-% umfasst.

11. Nutrazeutische Zusammensetzung für Hautpflege nach einem der vorstehenden Ansprüche, wobei die Zusammensetzung ferner Vitamin B9 in einem Bereich von 0,01 Gew.-% bis 0,05 Gew.-% umfasst.

12. Nutrazeutische Zusammensetzung für Hautpflege nach einem der vorstehenden Ansprüche, wobei die Zusammensetzung ferner L-Selenomethionin in einem Bereich von 0,38 Gew.-% bis 1 Gew.-% umfasst.

13. Nutrazeutische Zusammensetzung für Hautpflege nach einem der vorstehenden Ansprüche, wobei die Zusammensetzung ferner Yuzu-Aroma in einem Bereich von 3,44 Gew.-% bis 5 Gew.-% umfasst.

14. Nutrazeutische Zusammensetzung für Hautpflege nach einem der vorstehenden Ansprüche, wobei die Zusammensetzung ferner Moringablattextrakt umfasst.

15. Nutrazeutische Zusammensetzung für Hautpflege nach einem der vorstehenden Ansprüche, wobei die Zusammensetzung ferner Bärentraubenextrakt umfasst.

16. Nutrazeutische Zusammensetzung für Hautpflege nach einem der vorstehenden Ansprüche, wobei die Zusammensetzung ferner Kurkumaextrakt umfasst.

17. Nutrazeutische Zusammensetzung für Hautpflege nach einem der vorstehenden Ansprüche, wobei die Zusammensetzung ferner Wurzelextrakt von Picrorhiza kurroa Royle ex Benth umfasst.

18. Nutrazeutische Zusammensetzung für Hautpflege nach einem der vorstehenden Ansprüche, wobei die Zusammensetzung ferner Cholin umfasst.

19. Nutrazeutische Zusammensetzung für Hautpflege nach einem der vorstehenden Ansprüche, wobei die Zusammensetzung ferner *Tremella fuciformis* umfasst.

20. Nutrazeutische Zusammensetzung für Hautpflege nach einem der vorstehenden Ansprüche, wobei die Probiotika mit einer enterischen Beschichtung überzogen sind.

21. Nicht-therapeutische Verwendung einer nutrazeutischen Zusammensetzung nach einem der vorstehenden Ansprüche für Hautpflege, vorzugsweise um gesunde Hautfunktionen zu verbessern und zu fördern, die Hautelastizität zu verbessern, die Kollagensynthese zu steigern, das Erscheinen ungleichmäßiger Pigmentierung und Textur zu verringern, Haut zu hydratisieren und/oder dem Erscheinen von Fältchen und feinen Linien auf der Haut vorzubeugen.

22. Nutrazeutische Zusammensetzung nach einem der Ansprüche 1 bis 20 zur Verwendung bei der Reduzierung von Entzündungen.

23. Kosmetische Pflegemethode, umfassend die orale Einnahme einer nutrazeutischen Zusammensetzung für Hautpflege nach einem der Ansprüche 1 bis 20.

## Revendications

1. Composition nutraceutique pour le soin de la peau, comprenant :
- Inuline organique et oligofructose dans un rapport 2:1 dans une plage de 34,4 % à 55 % en poids ;
- Probiotiques dans une plage de 13,76 % à 30 % en poids ;
- L-Citrulline dans une plage de 17,20 % à 25 % en poids ;
- Extraits de grenade dans une plage de 8,60 % à 20 % en poids ;
- Extrait d'écorce de pin soluble à 98 % dans une plage de 2,41 % à 6 % en poids ;
- Extrait de racine de betterave biologique dans une plage de 3,44 % à 10 % en poids ;
- L-Arginine dans une plage de 1,72 % à 3 % en poids ;
- Extrait d'écorce de pastèque biologique dans une plage de 0,69 % à 10 % en poids ;
- Vitamine K2 dans une plage de 0,28 % à 2 % en poids ;
- Vitamine D3 dans une plage de 0,10 % à 0,45 % en poids.

2. Composition nutraceutique pour le soin de la peau selon la revendication 1, dans laquelle les probiotiques sont choisis parmi Lactobacillus acidophilus, Lactobacillus delbrueckii subsp. Bulgaricus, Streptococcus thermophilus, Lactococcus lactis ssp. Cremoris, Lactobacillus helveticus et une combinaison de ceux-ci.

3. Composition nutraceutique pour le soin de la peau selon l'une quelconque des revendications précédentes, dans laquelle les extraits de grenade comprennent punicalagines, ellagitanin, acide punicique ou une combinaison de ceux-ci.

4. Composition nutraceutique pour le soin de la peau selon l'une quelconque des revendications précédentes, dans laquelle la composition comprend en outre de la lactoferrine dans une plage de 3,44 % à 7 % en poids.

5. Composition nutraceutique pour le soin de la peau selon l'une quelconque des revendications précédentes, dans laquelle la composition comprend en outre de la vitamine B3 dans une plage de 0,55 à 1 % en poids.

6. Composition nutraceutique pour le soin de la peau selon l'une quelconque des revendications précédentes, dans laquelle la composition comprend en outre de la vitamine B5 dans une plage de 0,23 % à 1 % en poids.

7. Composition nutraceutique pour le soin de la peau selon l'une quelconque des revendications précédentes, dans laquelle la composition comprend en outre de la vitamine B6 dans une plage de 0,05 % à 1 % en poids.

8. Composition nutraceutique pour le soin de la peau selon l'une quelconque des revendications précédentes, dans laquelle la composition comprend en outre un extrait d'Ashwagandha dans une plage de 3,44 % à 15 % en poids.

9. Composition nutraceutique pour le soin de la peau selon l'une quelconque des revendications précédentes, dans laquelle la composition comprend en outre de la vitamine C dans une plage de 4,13 % à 15 % en poids.

10. Composition nutraceutique pour le soin de la peau selon l'une quelconque des revendications précédentes, dans laquelle la composition comprend en outre du bisglycinate de zinc dans une plage de 1,72 % à 5 % en poids.

11. Composition nutraceutique pour le soin de la peau selon l'une quelconque des revendications précédentes, dans laquelle la composition comprend en outre de la vitamine B9 dans une plage de 0,01 % à 0,05 % en poids.

12. Composition nutraceutique pour le soin de la peau selon l'une quelconque des revendications précédentes, dans laquelle la composition comprend en outre de la L-Sélénométhionine dans une plage de 0,38 % à 1 % en poids.

13. Composition nutraceutique pour le soin de la peau selon l'une quelconque des revendications précédentes, dans laquelle la composition comprend en outre un arôme de yuzu dans une plage de 3,44 % à 5 % en poids.

14. Composition nutraceutique pour le soin de la peau selon l'une quelconque des revendications précédentes, dans laquelle la composition comprend en outre un extrait de feuille de moringa.

15. Composition nutraceutique pour le soin de la peau selon l'une quelconque des revendications précédentes, dans laquelle la composition comprend en outre un extrait de busserole.

16. Composition nutraceutique pour le soin de la peau selon l'une quelconque des revendications précédentes, dans laquelle la composition comprend en outre un extrait de curcuma.

17. Composition nutraceutique pour le soin de la peau selon l'une quelconque des revendications précédentes, dans laquelle la composition comprend en outre un extrait de racine de Picrorhiza kurroa Royle ex Benth.

18. Composition nutraceutique pour le soin de la peau selon l'une quelconque des revendications précédentes, dans laquelle la composition comprend en outre de la choline.

19. Composition nutraceutique pour le soin de la peau selon l'une quelconque des revendications précédentes, dans laquelle la composition comprend en outre du *Tremella fuciformis.*

20. Composition nutraceutique pour le soin de la peau selon l'une quelconque des revendications précédentes, dans laquelle les probiotiques sont revêtus d'un revêtement entérique.

21. Utilisation non thérapeutique d'une composition nutraceutique selon l'une quelconque des revendications précédentes pour le soin de la peau, de préférence pour améliorer et stimuler la fonction saine de la peau, améliorer l'élasticité de la peau, augmenter la synthèse de collagène, réduire l'apparence de pigmentation et de texture inégales, hydrater la peau, et/ou prévenir l'apparition de rides et de ridules sur la peau.

22. Composition nutraceutique selon l'une quelconque des revendications 1 à 20, pour une utilisation dans la réduction d'une inflammation.

23. Procédé de soin cosmétique comprenant la prise orale d'une composition nutraceutique pour le soin de la peau selon l'une quelconque des revendications 1 à 20.
